# EUROPEAN PATENT APPLICATION

(11) **EP 2 716 264 A1**
(43) Date of publication of application: **09.04.2014**
(21) Application number: 12187148.7
(22) Date of filing: 04.10.2012
(51) Int. Cl.: A61F 5/00

(54) **Adjustable gastric band for laparoscopic gastric restrictive surgery**

(71) Applicant: Huang, Chih-Kun, Kaohsiung City 840 (TW)
(72) Inventor: Huang, Chih-Kun, Kaohsiung City 840 (TW)
(74) Representative: Dossmann, Gérard

(57) **Abstract**

A gastric band for laparoscopic gastric restrictive surgery includes a band (1) having a latch portion (11) and a latch element (12) which is fixed to the latch portion (11) when the band is encircled. A filler bag (2) is disposed on the inner circumferential surface of the band (1). A protecting saddle (3) extends axially from the lateral end of the band (1) and is located corresponding to the filler bag (2). For gastric band plicaton, the stomach is plicated to form a large gastric pouch and a plicated portion. For laparoscopic sleeve gastrectomy, a part of the stomach is removed to form the stomach to be a small sleeve shape, and the incision location is sewed. The filler bag (2) is covered below the connecting position of the patient's stomach and esophagus by the band (1), and the protecting saddle (3) is covered onto the plicated portion or incision location to prevent the patient from overeating.

## Description

The present invention relates to an adjustable gastric band for laparoscopic gastric restrictive surgery, and more particularly to the gastric band with a protecting saddle axially extended from a lateral end of the band and installed corresponding to a filler bag to protect patients from having complications caused by the expansion of an upper end of a plicated portion or an remnant part of the patient's stomach through overeating after the patient has taken a gastric plicaton or a laparoscopic sleeve gastrectomy, and the gastric band can achieve the effects of preventing the plicated portion or incision location of the patient's stomach from being cracked open as well as preventing the band from slipping off.

In recent years, obesity becomes an increasingly serious problem all over the world, and most patients cannot be cured by medicines or diet control only, and it is necessary to perform a surgery to inhibit the patients' food intake in order to achieve the weight reduction effect. Gastric restrictive surgery is one of the major technical means for treating obesity, and its main purpose resides on reducing the capacity of a patient's stomach, so that the patient can achieve sustained weight loss by limiting food intake, reducing appetite and slowing digestion. Present existing gastric restrictive surgeries are mainly divided into laparoscopic gastric plication and sleeve gastrectomy.

However, the gastric plication has a high rate of weight regain from dilatation of plicated stomach, and reports show that approximately 50% of the patients regain weight within five years after taking the surgery. And laparoscopic sleeve gastrectomy incurs a higher risk and the surgery is irreversible, such that the removed part cannot be restored. Although the aforementioned surgeries generally have a significant weight loss effect in a certain period of time, yet the stomach capacity will expand within a few years after the surgery. The issue of weight regain always frustrates medical professionals, and even worse requires the patients to take a second surgery.

In another conventional laparoscopic weight loss surgery, an adjustable gastric band is tied around a patient's upper gastric pouch for controlling the patient's food intake. Such gastric band can limit the patient's food intake only, but the stomach capacity is not reduced, and thus resulting in low weight loss rate, easy weight loss stagnation, and limited weight reduction efficiency. If the patient overeats, then complications such as dysphagia or slip-off of the gastric band may occur easily.

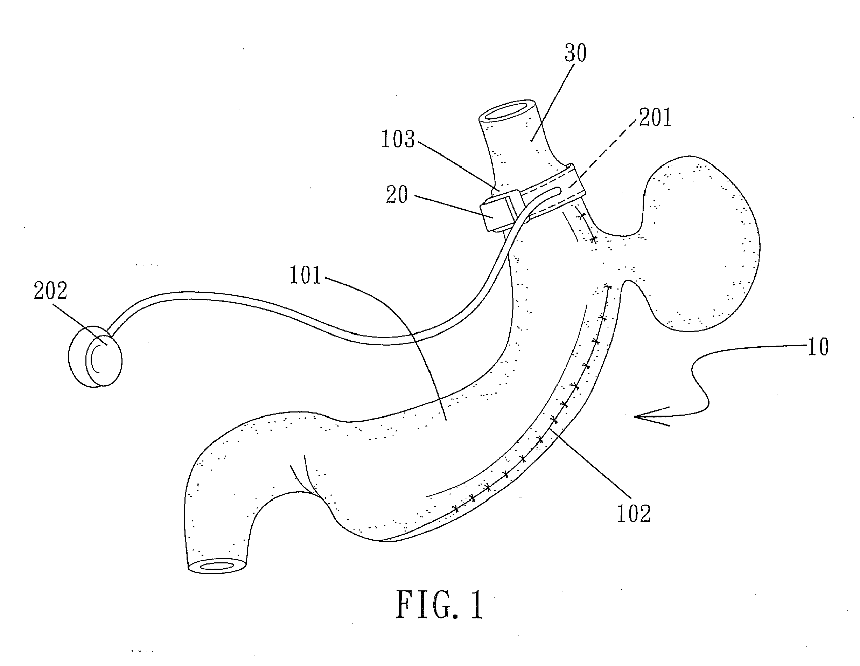

Therefore, the adjustable gastric banded plicaton was developed and introduced, and it combines the adjustable gastric band and the gastric plication surgery as shown in FIG. 1, wherein the stomach 10 is plicated and sewed to form a long gastric tube 101 and a plicated portion 102, so that the stomach capacity drops to approximately 100mL, and a conventional adjustable gastric band is adopted, and this band 20 is made of silicone and can be encircled. The band 20 includes a filler bag 201 installed on an inner circumferential surface of the band 20 and coupled to a fluid injection port 202, such that the filler bag 201 can be tied below the connecting position of the stomach 10 and an esophagus 30 through the band 20, and the stomach 10 forms a small gastric pouch 103 of approximately 25mL, and the fluid injection port 202 is provided for injecting a fluid into the filler bag 201 to adjust the tightness of tying the band 20, so as to control the patient's food intake and enhance the weight reduction effect. In addition, the gastric banded plicaton is reversible and capable of improving the weight reduction efficiency, reducing the revisit frequency effectively, and overcoming the problem of the patient having poor absorption of vitamins after taking the conventional gastric bypass surgery.

However, the adjustable gastric band 20 is not specifically designed for the adjustable gastric banded plicaton surgery, and the filler bag 201 is simply tied below the connecting position of the stomach 10 and the esophagus 30 after the gastric band plicaton takes place. However, if a patient overeats, then the stomach 10 will expand to bulge out an upper end of the plicated portion 101 and cause complications, or crack open the plicated portion 101, so that the band 20 may slip off easily by the expansion of the stomach 10. As a result, such conventional band 20 jeopardizes the patient's health and life safety.

In addition, the conventional laparoscopic sleeve gastrectomy changes the stomach in the original pouch shape into a stomach in a small sleeve shape by removing part of greater curvature of the stomach through surgery. In adjustable banded sleeve gastrectomy, the filler bag 201 is tied below the connecting position of the stomach 10 and the esophagus 30 through the band 20, and the fluid injection port 202 is used for injecting a fluid into the filler bag 201 to adjust the tightness of tying the band 20 in order to control the patient's food intake. However, the band 20 is not applicable for laparoscopic sleeve gastrectomy, and the filler bag 201 is simply tied below the connecting position of the stomach 10 and the esophagus 30 after the laparoscopic sleeve gastrectomy takes place. However, if the patient overeats, then the band 20 will slip off easily by the expansion of the stomach 10, and an upper end of the incision portion will expand and bulge to cause complications or the incision location is cracked open, and thus the risk is even higher than the conventional adjustable gastric band applied in gastric band plicaton. Obviously, such conventional band 20 substantially jeopardizes the patient's health and life safety.

In view of the aforementioned drawbacks of the prior art, the inventor of the present invention based on years of experience in the related field to conduct extensive researches and experiments to improve the gastric band structure, and finally developed a gastric band of the invention to overcome the drawbacks of the prior art.

Therefore, it is a primary objective of the present invention to overcome the drawbacks of the conventional gastric band that is not specifically designed for gastric band plicaton and laparoscopic sleeve gastrectomy, such that if the patient overeats, the stomach pouch will be expanded to bulge the upper part of the plicated portion or incision location and cause complications easily or crack open the incision location, and the band may slip off easily due to the expansion of the stomach, and thus jeopardizing the patient's health and life safety.

To achieve the foregoing objective, the present invention provides an adjustable gastric band for laparoscopic gastric restrictive surgery: gastric plication or sleeve gastrectomy, comprising: a band, having a latch portion formed at an end, and a latch element installed at the other end and corresponding to the latch portion, such that the latch portion and the latch element are fixed with each other when the band is encircled; a filler bag, disposed on an inner circumferential surface of the band; and a protecting saddle, extended axially from a lateral end of the band and corresponding to the filler bag.

In the aforementioned adjustable gastric band for laparoscopic gastric restrictive surgery, the protecting saddle further comprises at least one through hole.

In the aforementioned adjustable gastric band for laparoscopic gastric restrictive surgery, the latch element further comprises a fastening element and a connecting portion corresponding to the fastening element and the latch portion, and the band includes a collar, and the fastening element is installed at the collar, and the connecting portion is disposed on an outer surface of the band and corresponding to the collar, thereby the latch portion is passed through the collar, and the fastening element is provided for latching the latch portion to the connecting portion when the band is encircled.

The aforementioned adjustable gastric band for laparoscopic gastric restrictive surgery further comprises an injector coupled to the filler bag for injecting a fluid to control an expanding/contracting status of the filler bag.

In the aforementioned adjustable gastric band for laparoscopic gastric restrictive surgery, the band is a silicone band.

With the foregoing assembly, the present invention can be applied in gastric banded plicaton and laparoscopic banded sleeve gastrectomy. For applications in the gastric banded plicaton, the stomach is plicated to form a plicated gastric tube; and for application in the laparoscopic sleeve gastrectomy, a part of the stomach is removed to form a stomach in a small sleeve shape, and the incision location is sewed, and then the band is tied below the connecting position of the patient's stomach and esophagus through the filler bag, and the protecting saddle covers the corresponding plicated portion or incision location of the patient's stomach, wherein the protecting saddle has through holes for fixing the protecting saddle onto a surface of upper gastric tube to prevent the plicated portion or incision location of the patient's stomach from being cracked open and preventing the band from slipping off. In addition, the band of the present invention is tied at the upper gastric tube to form a small gastric pouch, and the injector is used for adjusting the tightness of the filler bag to encicle the stomach pouch, while controlling the patient's food intake. The invention has the advantage and effect of improving the surgical safety and applicability.

FIG. 1 is a schematic view of a conventional adjustable gastric band used in a gastric band plicaton;

FIG. 2 is a perspective view of an untied gastric band of the present invention;

FIG. 3 is a cross-sectional view of Section A-A as depicted in FIG. 2;

FIG. 4 is a perspective view of a tied gastric band of the present invention;

FIG. 5 is a cross-sectional view of Section B-B as depicted in FIG. 4;

FIG. 6 is a schematic view of a stomach to be plicated; and

FIG. 7 is a schematic view of an application status of the present invention.

The technical characteristics of the present invention will become apparent with the detailed description of preferred embodiments and the illustration of related drawings as follows.

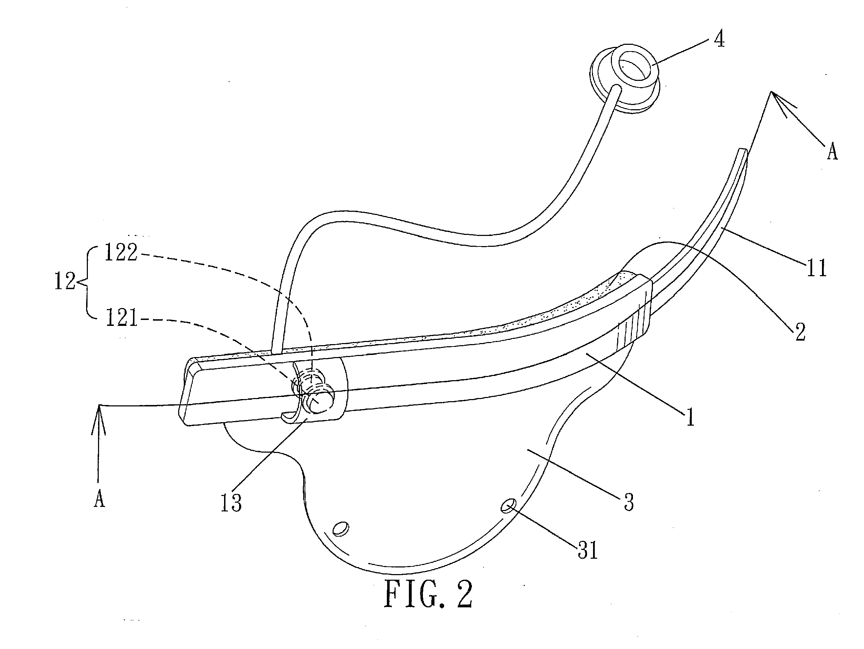

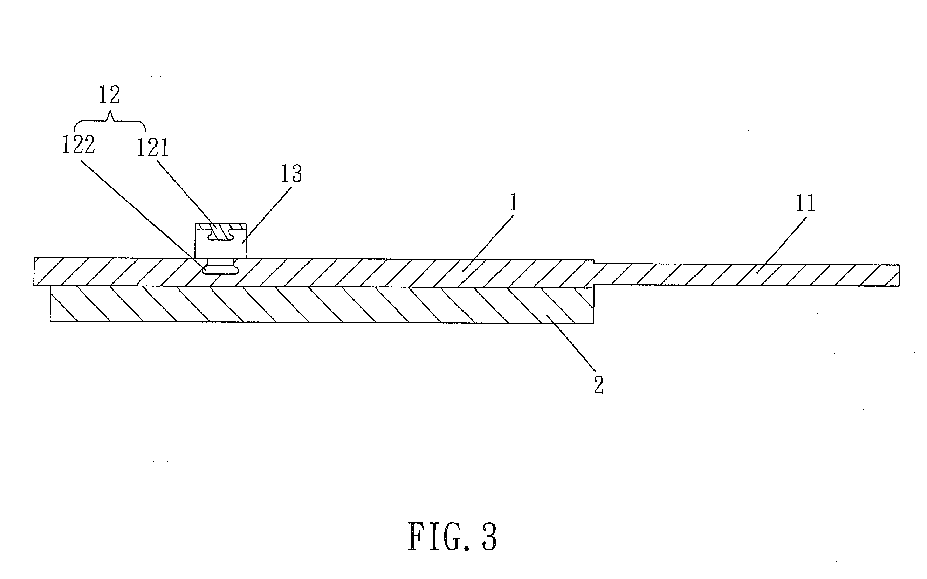

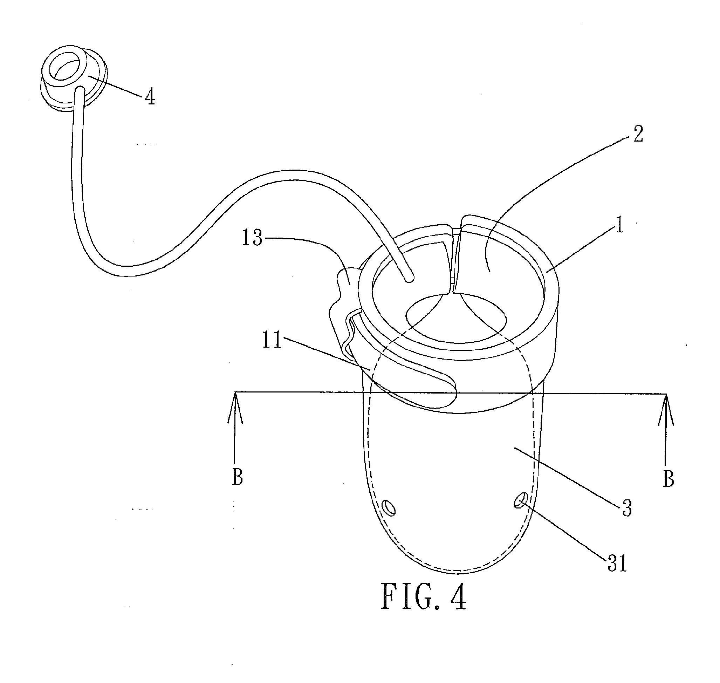

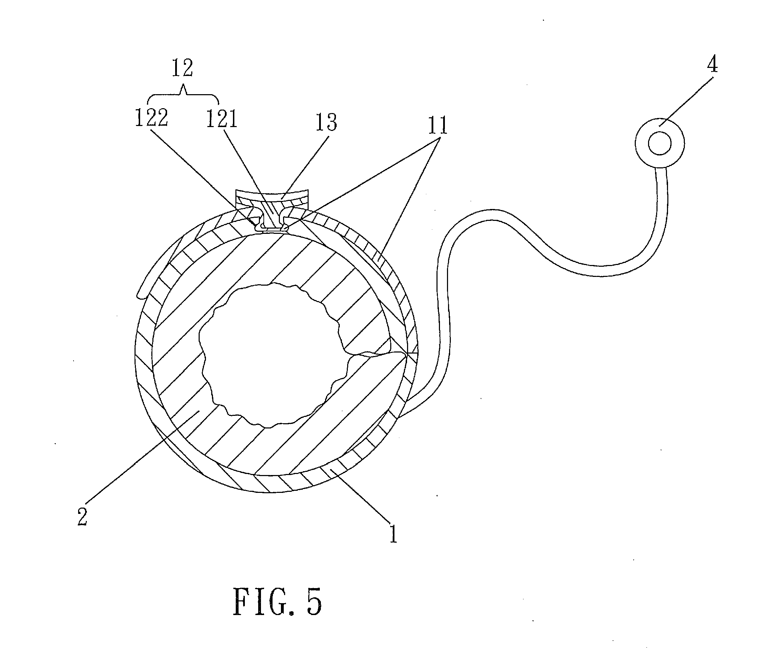

With reference to FIGS. 2 to 7 for an adjustable gastric band for laparoscopic gastric restrictive surgery in accordance with the present invention, the gastric band comprises: a band 1, which is a silicone band, having a latch portion 11 formed at an end of the band 1; a latch element 12 installed at the other end of the band 1 and corresponding to the latch portion 11, such that the latch portion 11 and the latch element 12 are fixed with each other when the band 1 is encircled, and the latch element 12 further comprises a fastening element 121 and a connecting portion 122 corresponding to the fastening element 121 and the latch portion 11, and the band 1 has a collar 13 extended from the band 1, and the fastening element 121 is installed at the collar 13, and the connecting portion 122 is installed on an outer surface of the band 1 and corresponding to the collar 13; so that when the band 1 is encircled, the latch portion 11 is passed through the collar 13, and the fastening element 121 latches the corresponding latch portion 11 to the connecting portion 122, and the band 1 includes a filler bag 2 disposed on an inner circumferential surface of the band 1; a protecting saddle 3 extended axially from a lateral end of the band 1 and corresponding to the filler bag 2, and the protecting saddle 3 further includes at least one through hole 31; and an injector 4, coupled to the filler bag 2 for injecting a fluid to control the expanding/contracting status of the filler bag 2.

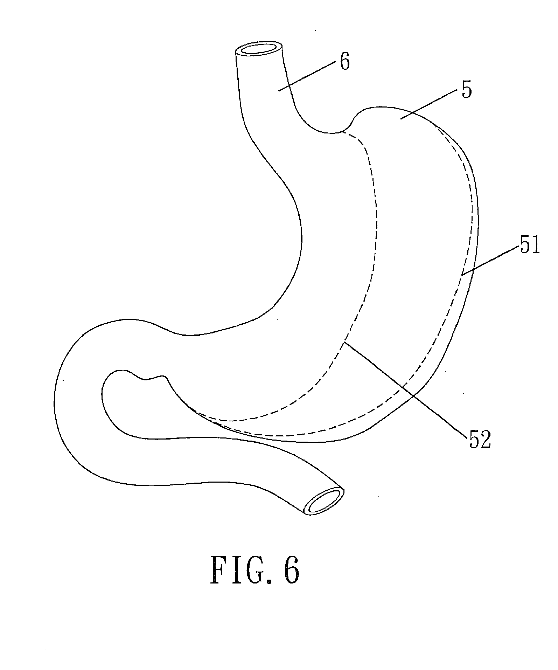

With reference to FIG. 6 for an operation of the present invention, a first plication line 51 for a gastric plication of the stomach 5 is planned for the stomach 5 and the esophagus 6 before the gastric band plicaton is performed, and the stomach 5 is inwardly collapsed along the first plication line 51 through the first plication line 51 and two symmetric second plication lines 52, and a thread 7 is used for sewing the second plication lines 52 together, so that stomach 5 forms a large gastric pouch 53 with a capacity of approximately 100mL and a plicated portion 54.

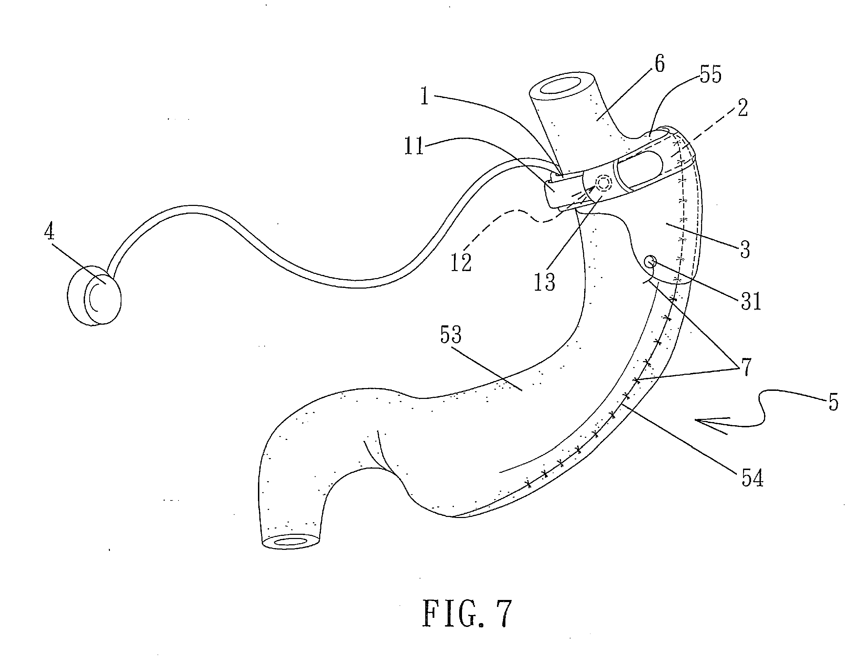

With reference to FIG. 7, the band 1 of the present invention is used to tie the filler bag 2 below the connecting position of the stomach 5 and the esophagus 6 at the plicated and sewed stomach 5, and the latch portion 11 is passed through the collar 13, and the fastening element 121 latches the latch portion 11 into the connecting portion 122 to fix the band 1, so that the stomach 5 forms a small gastric pouch 55 of approximately 25mL, and the injector 4 is used for injecting a fluid into the filler bag 2 to control the expanding/contracting status of the filler bag 2 in order to achieve the effects of adjusting the tightness of tying the band 1 to the stomach 5, and limiting the patient's food intake to enhance the weight reduction efficiency. The protecting saddle 3 is then covered onto the plicated portion 54 to prevent the patient from overeating which may expand and bulge the upper end of the plicated portion 54 due to the expansion of the stomach and cause complications, and also prevent the plicated portion 54 from being cracked open by the expansion of the stomach. The protecting saddle 3 includes a through hole 31, and a thread 7 is passed through the through hole 31 to enhance the fixture of the protecting saddle 3 at the large gastric pouch 53 and prevent the band 1 from slipping off by the expansion of the stomach. Obviously, the present invention can improve the safety after the gastric band plicaton takes place and achieve the effects of enhancing the weight reduction efficiency, reducing the revisit frequency, and lowering the probability of having complications.

With reference to FIG. 7, if it is necessary to remove the gastric band of the present invention after the surgery takes place, the thread 7 in the through hole 31 can be removed easily, and the fastening element 121 is removed from the connecting portion 122, so that the latch portion 11 can pull out the collar 13 to complete the process of removing the band 1, and thus the present invention provides convenient installation and removal.

The present invention also can be used in laparoscopic sleeve gastrectomy (not shown in the figure), wherein a part of the stomach is removed to form a stomach in a small sleeve shape, and the incision location is sewed, and the band 1 is used to tie the filler bag 2 around a position below the connecting position of the stomach 5 and the esophagus 6, and the protecting saddle 3 is covered onto the corresponding incision location to prevent the patient from overeating which may expand and bulge the upper end of the incision location due to the expansion of the stomach and cause complications, and also prevent the incision location from being cracked open by the expansion of the stomach. The protecting saddle 3 includes a through hole 31, and a thread 7 is passed through the through hole 31 to enhance the fixture of the protecting saddle 3 at the large gastric pouch 53 and prevent the band 1 from slipping off by the expansion of the stomach.

The method of operating the gastric band of the present invention in laparoscopic sleeve gastrectomy is the same as that of the gastric band plicaton, and thus will not be repeated.

The description and structure of the Obviously, the present invention can improve the safety after the gastric band plicaton takes place and achieve the effects of enhancing the weight reduction efficiency, reducing the revisit frequency, and lowering the probability of having complications.

In summation of the description above, the present invention obviously has one or more of the following advantages and effects:

1. The present invention can be applied in both gastric band plicaton and laparoscopic sleeve gastrectomy to improve the scope of applicability of the present invention, and the present invention can inject a fluid into the filler bag 2 through the injector 4 to adjust the tightness of tying the band 1 to the patient's stomach 5, so as to limit and control the patient's food intake to improve the weight reduction effect, and also provides a protecting saddle 3 covered onto the plicated portion 54 or incision location to prevent the patient from overeating which may expand and bulge the upper end of the plicated portion 54 due to the expansion of the stomach and cause complications, and also prevent the plicated portion 54 or incision location from being cracked open by the expansion of the stomach. Thus, the present invention can improve the safety after the gastric band plicaton or laparoscopic sleeve gastrectomy takes place and can achieve the effects of enhancing the weight reduction efficiency, reducing the revisit frequency, and lowering the probability of having complications.

2. The protecting saddle 3 includes a through hole 31, and a thread 7 is passed through the through hole 31 to enhance the fixture of the protecting saddle 3 at the large gastric pouch 53 and improve the effect of the protecting saddle 3 covered onto he plicated portion 54 or incision location, so as to prevent the band 1 from slipping off by the expansion of the stomach. Thus, the invention can achieve the effect of improving the safety and stability after the gastric band plicaton and laparoscopic sleeve gastrectomy takes place.

3. If it is necessary to remove the gastric band of the present invention after the surgery takes place, the thread 7 in the through hole 31 can be removed easily, and the fastening element 121 is removed from the connecting portion 122, so that the latch portion 11 can pull out the collar 13 to complete the process of removing the band 1, and thus the present invention provides convenient installation and removal.

While we have shown and described the embodiment in accordance with the present invention, it should be clear to those skilled in the art that further embodiments may be made without departing from the scope of the present invention.

## Claims

1. An adjustable gastric band for laparoscopic gastric restrictive surgery, comprising:
a band having a latch portion formed at an end and a latch element installed at the other end and located corresponding to the latch portion, such that the latch portion and the latch element are fixed with each other when the band is encircled;
a filler bag disposed on an inner circumferential surface of the band, and
a protecting saddle extended axially from a lateral end of the band and located corresponding to the filler bag.

2. The adjustable gastric band for laparoscopic gastric restrictive surgery as claimed in claim 1, wherein the protecting saddle further comprises at least one through hole.

3. The gastric band for laparoscopic gastric restrictive surgery as claimed in claim 1, wherein the latch element comprises a fastening element and a connecting portion corresponding to the fastening element and the latch portion, the band includes a collar, the fastening element is installed at the collar, the connecting portion is disposed on an outer surface of the band and corresponding to the collar, the latch portion is passed through the collar and the fastening element is provided for latching the latch portion to the connecting portion when the band is encircled.

4. The adjustable gastric band for laparoscopic gastric restrictive surgery as claimed in claim 2, wherein the latch element comprises a fastening element and a connecting portion corresponding to the fastening element and the latch portion, the band includes a collar, the fastening element is installed at the collar, the connecting portion is disposed on an outer surface of the band and corresponding to the collar, the latch portion is passed through the collar and the fastening element is provided for latching the latch portion to the connecting portion when the band is encircled.

5. The adjustable gastric band for laparoscopic gastric restrictive surgery as claimed in claim 1, further comprising an injector coupled to the filler bag for injecting a fluid to control an expanding/contracting status of the filler bag.

6. The adjustable gastric band for laparoscopic gastric restrictive surgery as claimed in claim 2, further comprising an injector coupled to the filler bag for injecting a fluid to control an expanding/contracting status of the filler bag.

7. The adjustable gastric band for laparoscopic gastric restrictive surgery as claimed in claim 1, wherein the band is a silicone band.

8. The adjustable gastric band for laparoscopic gastric restrictive surgery as claimed in claim 2, wherein the band is a silicone band.

## Amended claims

### Amended claims in accordance with Rule 137(2) EPC.

**1.** An adjustable gastric band for laparoscopic gastric restrictive surgery, comprising:
a band (1) having a latch portion (11) formed at an end and a latch element (12) installed at the other end and located corresponding to the latch portion (11), such that the latch portion (11) and the latch element (12) are fixed with each other when the band (1) is encircled;
a filler bag (2) disposed on an inner circumferential surface of the band (1), and
a protecting saddle (3) extended axially from a lateral end of the band (1) and located corresponding to the filler bag (2),
**characterized in that**
the latch element (12) comprises a fastening element (121) and a connecting portion (122) corresponding to the fastening element (121) and the latch portion (11), the band (1) includes acollar (13), the fastening element (121) is installed at the collar (13), the connecting portion (122) is disposed on an outer surface of the band (1) and
corresponding to the collar (13), the latch portion (11) is passed through the collar (13) and the fastening element (121) is provided for latching the latch portion (11) to the connecting portion (122) when the band (1) is encircled.

**2.** The adjustable gastric band for laparoscopic gastric restrictive surgery as claimed in claim 1, wherein the protecting saddle (3) further comprises at least one through hole (31).

**3.** The adjustable gastric band for laparoscopic gastric restrictive surgery as claimed in claim 1, further comprising an injector (4) coupled to the filler bag for injecting a fluid to control an expanding/contracting status of the filler bag (2).

**4.** The adjustable gastric barid for laparoscopic gastric restrictive surgery as claimed in claim 1, wherein the band (1) is a silicone band.
